# FASCICULE DE BREVET EUROPEEN

(11) **EP 2 601 918 B1**
(45) Date de publication et mention de la délivrance du brevet: **04.06.2014**
(21) Numéro de dépôt: 12196082.7
(22) Date de dépôt: 07.12.2012
(51) Int. Cl.: A61F 9/007, A61N 7/00

(54) **Dispositif de succion pour le traitement d'une pathologie oculaire**
Saugvorrichtung für die Behandlung einer Augenkrankheit
Suction device for treating an ocular disease

(30) Priorité: 08.12.2011 FR 1161311
(43) Date de publication de la demande: 12.06.2013
(73) Titulaire: Eye Tech Care, 69140 rillieux-la-Pape (FR)
(72) Inventeur: Romano, Fabrizio, 01700 BEYNOST (FR); Chapuis, Philippe, 69480 POMMIERS (FR); Farcy, Laurent, 69400 LIERGUES (FR)
(74) Mandataire: Regimbeau

(56) Documents cités:
- WO-A1-96/14019
- WO-A1-2010/094353
- WO-A1-2011/083358
- US-A1- 2003 114 861
- US-A1- 2011 190 741

## Description

### DOMAINE TECHNIQUE DE L'INVENTION

La présente invention concerne le domaine technique général des dispositifs de traitement d'une pathologie oculaire, comme par exemple le traitement d'un glaucome.

### ARRIERE-PLAN DE L'INVENTION

Certaines interventions chirurgicales sur l'oeil d'un patient nécessitent l'utilisation d'un anneau de succion, tel que décrit dans le document EP 1 243 236.

C'est notamment le cas des chirurgies oculaires pour le traitement d'une myopie qui consiste à utiliser des instruments comprenant un anneau de succion, un instrument à microkératome et un laser.

Le principe d'utilisation de ces instruments dans le cas du traitement d'une myopie est le suivant.

L'anneau est disposé sur l'oeil du patient et un vide d'air est créé, en activant par exemple une pompe à vide connectée à l'anneau de succion. L'aspiration exercée par l'anneau de succion provoque une déformation de la surface de l'oeil qui tend à s'aplatir. Lorsque le site cornéen est convenablement préparé (i.e. surface de l'oeil suffisamment aplatie), le praticien débute l'intervention chirurgicale.

Un microkératome est positionné sur l'anneau de succion. Le microkératome est ensuite déplacé en translation pour couper un lambeau cornéen, lequel est ensuite retourné.

Un laser positionné au dessus de la zone à traiter est activé pour exciser le fragment exposé de la cornée et obtenir la correction de la vision recherchée.

Le praticien replace ensuite le lambeau cornéen dans sa position initiale.

L'utilisation d'un anneau de succion de ce type pour réaliser une telle intervention chirurgicale du fait de la force d'aspiration considérable et du positionnement de l'anneau en regard d'une zone, le limbe, non recouverte par la membrane conjonctive, induit une augmentation de la pression intraoculaire du patient, celle-ci pouvant atteindre 400 millimètres de mercure ou plus.

Pour certaines interventions chirurgicales, par exemple dans le cas du traitement de patient glaucomateux, une telle augmentation de la pression intraoculaire n'est pas acceptable.

Le document W02010/094353 décrit un dispositif de traitement d'une pathologie oculaire comportant un anneau oculaire et des moyens de génération d'ultrason focalisés de haute intensité fixés à l'extrémité distale de l'anneau. L'extrémité proximale de l'anneau est adaptée pour être appliquée sur le globe oculaire du patient à traiter. L'anneau oculaire peut être raccordé à un dispositif d'aspiration.

Un but de la présente invention est de proposer un anneau de succion pour le traitement d'une pathologie oculaire permettant de limiter l'augmentation de la pression intraoculaire.

### BREVE DESCRIPTION DE L'INVENTION

A cet effet l'invention propose un dispositif de traitement d'une pathologie oculaire comportant :
- un anneau de succion notamment conique incluant une extrémité proximale adaptée pour venir en butée contre l'oeil d'un patient et comportant une portion creuse,
- un système d'aspiration connecté à la portion creuse pour aspirer l'air dans la portion creuse de façon à fixer l'anneau sur l'oeil du patient,
remarquable en ce que le système d'aspiration est adapté pour générer une dépression légère comprise entre 80 et 150 millimètres de mercure (mmHg), et préférentiellement comprise entre 120 et 150mmHg.

De préférence, la portion creuse est inscrite sur un cercle dont le centre appartient à l'axe de symétrie de l'anneau et dont le diamètre est compris entre 14 et 24 millimètres, de préférence compris entre 14 et 22 millimètres, et encore plus préférentiellement compris entre 14 et 18 millimètres, de sorte que lorsque l'anneau est en position sur l'oeil (c'est-à-dire que l'anneau est correctement positionné sur l'oeil, ou encore que l'anneau est centré sur l'oeil, ou encore que l'axe optique de l'oeil et un axe de symétrie de l'anneau sont coaxiaux) la portion creuse est en regard d'une zone de l'oeil où la sclérotique est recouverte d'une fine membrane conjonctivale.

Le fait que la zone d'aspiration soit placée non pas en regard du limbe dépourvu de conjonctive, mais à environ 2 mm au-delà de celui-ci, soit en regard d'une zone où la sclérotique est recouverte d'une fine membrane conjonctivale permet d'obtenir une aspiration douce de la conjonctive bulbaire limitant l'augmentation de la pression intraoculaire de l'oeil tout en solidarisant l'anneau et l'oeil à traiter.

Des aspects préférés mais non limitatifs du dispositif selon l'invention sont les suivants :
- l'anneau comprend une ouverture d'où la cornée de l'oeil est projetée, et un berceau de support destiné à recevoir des moyens de génération d'ultrasons ;
- la portion creuse comprend au moins un évidement,
- la portion creuse comprend une pluralité d'évidements, chaque évidement étant connecté de manière indépendante ou non au système d'aspiration,
- la portion creuse comprend au moins une rainure,
- la portion creuse comprend une pluralité de rainures
- la portion creuse comprend une gorge annulaire,

### BREVE DESCRIPTION DES DESSINS

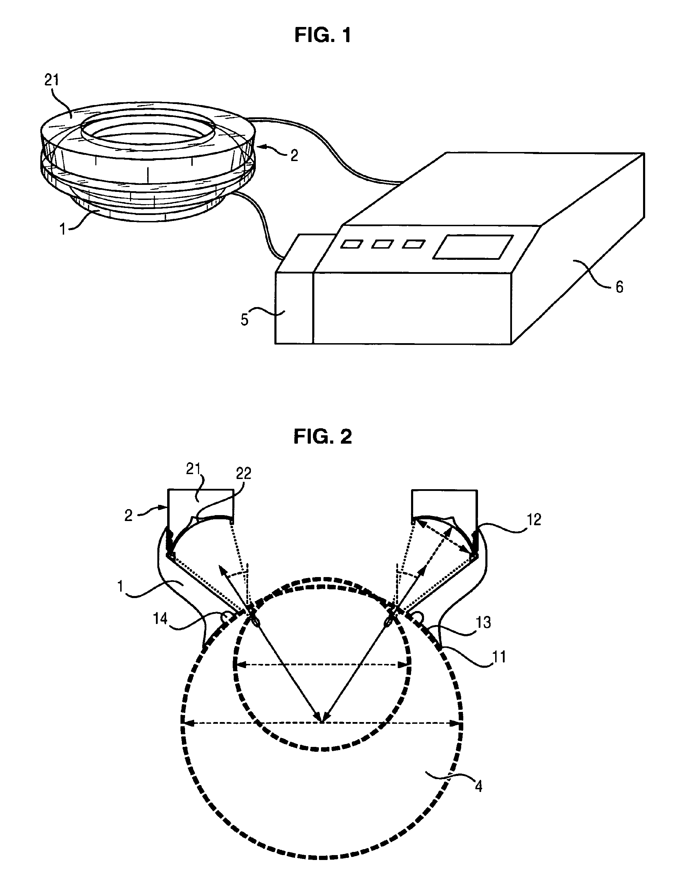

D'autres avantages et caractéristiques ressortiront mieux de la description qui va suivre de plusieurs variantes d'exécution, données à titre d'exemples non limitatifs, à partir des dessins annexés sur lesquels les figures 1 à 4 illustrent différents modes de réalisation du dispositif selon l'invention.

### DESCRIPTION DETAILLEE DE L'INVENTION

En référence aux figures 1 et 2, on a illustré un mode de réalisation du dispositif de traitement d'une pathologie oculaire.

Le dispositif comprend :
- un anneau 1,
- des moyens de génération d'ultrasons 2 pour générer des ultrasons, et
- un système d'aspiration 5.

L'anneau 1 consiste en un tronc de cône ouvert aux deux extrémités. La petite base 11 du tronc de cône constitue l'extrémité proximale de l'anneau 1, et la grande base 12 constitue l'extrémité distale de l'anneau 1.

L'anneau 1 permet un positionnement adéquat et constant des moyens de génération d'ultrasons 2, aussi bien pour le centrage que pour la distance par rapport à la sclère des moyens de génération d'ultrasons 2.

L'extrémité proximale 11 est destinée à venir en contact avec un oeil 4 d'un patient. L'extrémité distale 12 de l'anneau 1 comprend un berceau support destiné à recevoir les moyens de génération d'ultrasons 2.

En référence à la figure 1, l'extrémité proximale 11 du tronc de cône comprend une bride annulaire externe 13 apte à être appliquée sur la surface externe de l'oeil 4, à environ 2 mm du limbe, le limbe étant la jonction entre la cornée et la sclérotique.

La face proximale de la bride annulaire 13 présente un profil concave, le rayon de courbure du profil concave étant sensiblement égal au rayon de courbure de l'oeil 4.

Le bord proximal 11 du tronc de cône comporte également une portion creuse 14 reliée au dispositif d'aspiration 5 par au moins une ouverture traversant le tronc de cône 1 et débouchant dans la portion creuse 14. Avantageusement, le dispositif d'aspiration 5 peut être contrôlé par une unité de commande 6.

Il est évident que le dispositif d'aspiration 5 peut être indépendant sans sortir du cadre de l'invention.

La portion creuse 14 est positionnée sur l'anneau de sorte que, en utilisation (c'est à dire lorsque l'anneau est positionné sur l'oeil), cette portion creuse vienne en contact avec la membrane conjonctivale. Ceci permet de limiter les risques de déformation de l'oeil du fait de la dépression générée dans la portion creuse.

En effet, dans le cas des dispositifs de succion de l'art antérieur, la portion creuse est positionnée sur l'anneau de sorte à venir directement en contact avec la surface de l'oeil au niveau du limbe. L'application d'une dépression dans la portion creuse induit alors une déformation de l'oeil (notamment lorsque la dépression générée est importante) tendant à en augmenter la pression intraoculaire.

A l'inverse, dans le cas de la présente invention, la portion creuse est positionnée sur l'anneau de sorte à venir indirectement en contact avec la surface de l'oeil, la membrane conjonctivale s'étendant entre la portion creuse et la surface de l'oeil.

Ainsi, l'application d'une dépression dans la portion creuse du dispositif selon l'invention induit la déformation de la membrane conjonctivale. Celle-ci se tend et glisse sur l'oeil. Du fait de son coefficient d'élasticité non nul, la mise en tension de la membrane conjonctivale n'induit pas l'application de forces suffisantes sur l'oeil pour entraîner sa déformation. La pression intraoculaire dans l'oeil reste sensiblement invariante, même sous l'effet de la dépression appliquée par le dispositif de succion.

De préférence, la position de la portion creuse sur l'anneau est telle que celle-ci soit inscrite sur un cercle dont le centre appartient à l'axe de symétrie de l'anneau et dont le diamètre est compris entre 14 et 24 millimètres, de préférence compris entre 14 et 22 millimètres, et encore plus préférentiellement compris entre 14 et 18 millimètres. Ceci permet de garantir que lorsque l'anneau est positionné sur l'oeil, la portion creuse vienne en contact avec la membrane conjonctivale.

Lors de l'application d'une dépression, la membrane conjonctivale se déforme. Une partie de la membrane conjonctivale pénètre dans la portion creuse et tend à l'obstruer. Ainsi, le maintien en position du dispositif de succion est obtenu en « ventousant » sur la membrane conjonctivale dont l'élasticité importante permet de limiter les risques de déformation de l'oeil et donc d'augmentation de la pression intraoculaire de celui-ci.

La portion creuse 14 peut présenter différentes formes.

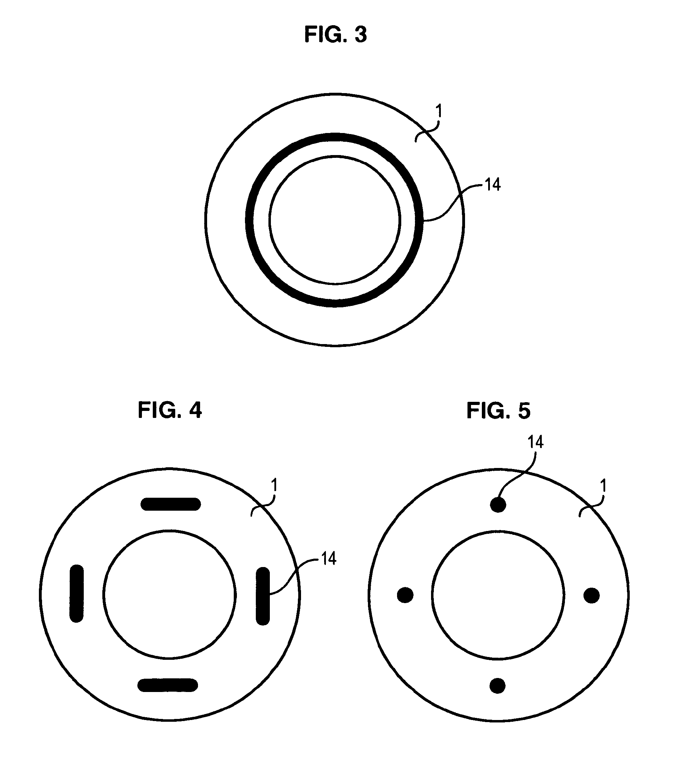

Dans le mode de réalisation illustré à la figure 3, la portion creuse consiste en une gorge annulaire. Ceci permet d'assurer un ventousage de l'anneau à l'oeil sur toute la surface de contact entre l'anneau et l'oeil.

Dans le mode de réalisation illustré à la figure 4, la portion creuse consiste en une pluralité (deux ou quatre) de rainures (figure 4) et/ou d'évidements (figure 5). Les rainures peuvent être sensiblement rectilignes et s'étendre le long d'un axe ou être incurvées et s'étendre le long d'une portion de cercle pour suivre la forme sphérique du globe oculaire.

Le fait que la portion creuse consiste en une pluralité (deux ou quatre) de rainures et/ou d'évidements permet de concentrer la force d'aspiration sur des zones réduites tout en limitant les risques de déformation de l'oeil du fait de l'aspiration.

Avantageusement, chaque évidement ou rainure peut être connecté indépendamment ou non au système d'aspiration. Ceci permet d'assurer une dépression uniforme au niveau de chacun des évidements ou de chacune des rainures.

Le système d'aspiration 5 comprend une ou plusieurs pompes à vide. Lorsque le système d'aspiration comprend plusieurs pompes à vide, chacune de celles-ci peut être connectée à un évidement respectif ou à une rainure respective.

Avantageusement, le système d'aspiration est adapté pour générer une dépression légère comprise entre 80 et 150 millimètres de mercure, de préférence égale à 120 millimètres de mercure.

Ceci permet d'obtenir une aspiration douce de la conjonctive bulbaire, solidarisant l'anneau et l'oeil à traiter tout en limitant la pression intraoculaire de l'oeil.

Le système d'aspiration peut comprendre une sonde permettant de détecter un paramètre oculaire correspondant à la pression intraoculaire, et étant capable de fournir des mesures de pression intraoculaire. Cette mesure de pression intraoculaire peut avantageusement être prise en compte par des moyens de contrôle du système d'aspiration afin de commander la ou les pompe à vide afin de maintenir la dépression à une valeur inférieure à 120 millimètres de mercure et préférentiellement comprise entre 80 et 100 millimètres de mercure.

Le principe de fonctionnement du dispositif d'aspiration 5 est le suivant. Le tronc de cône 1 est appliqué sur l'oeil 4 du patient et le dispositif d'aspiration 5 est activé. Ceci induit la production d'une dépression dans la gorge annulaire 14 qui entraine une déformation de la conjonctive de l'oeil 4, cette déformation formant un joint torique dans la gorge annulaire 14.

Le tronc de cône 1 est alors étroitement lié à l'oeil 4 de sorte que le tronc de cône 1 suivra les micromouvements de l'oeil 4 au cours de la durée du traitement. Ceci permet d'assurer un maintien de la position centrée de l'appareil sur l'axe visuel.

Après obtention du ventousage de l'anneau sur l'oeil, les moyens de génération d'ultrasons - composés de six transducteurs - peuvent être positionnés dans le berceau de support de l'anneau, et le praticien peut alors mettre en oeuvre le traitement désiré en bénéficiant d'une sécurité accrue étant donné que le système de ventousage par anneau de succion permet de maintenir la position de la sonde pendant toute la durée du traitement.

L'homme du métier aura compris que de nombreuses modifications peuvent être apportées au dispositif décrit ci-dessus sans sortir matériellement des nouveaux enseignements présentés ici. Il est donc bien évident que les exemples qui viennent d'être donnés ne sont que des illustrations particulières en aucun cas limitatives.

## Revendications

1. Dispositif de traitement d'une pathologie oculaire comportant :
- un anneau de succion (1) incluant une extrémité proximale adaptée pour venir en butée contre l'oeil d'un patient et comportant une portion creuse,
- un système d'aspiration (5) connecté à la portion creuse pour aspirer l'air dans la portion creuse de façon à fixer l'anneau sur l'oeil du patient,
**caractérisé en ce que** le système d'aspiration est adapté pour générer une dépression légère comprise entre 80 et 150 millimètres de mercure.

2. Dispositif selon la revendication 1, dans lequel la position de la portion creuse sur l'anneau est telle que lorsque l'anneau est en position sur l'oeil, la portion creuse est en contact avec une zone de l'oeil où la sclérotique est recouverte d'une fine membrane conjonctivale.

3. Dispositif selon l'une quelconque des revendications 1 à 2, dans lequel le système d'aspiration est adapté pour générer une dépression sensiblement égale à 120 millimètres de mercure.

4. Dispositif selon l'une quelconque des revendications 1 à 3, dans lequel l'anneau comprend une ouverture d'où la cornée de l'oeil est projetée, et un berceau de support destiné à recevoir des moyens de génération d'ultrasons.

5. Dispositif selon l'une quelconque des revendications 1 à 4, dans lequel la portion creuse comprend au moins un évidement.

6. Dispositif selon l'une quelconque des revendications 1 à 5, dans lequel la portion creuse comprend une pluralité d'évidements.

7. Dispositif selon la revendication 6, dans lequel chaque évidement est connecté de manière indépendante au système d'aspiration.

8. Dispositif selon l'une quelconque des revendications 1 à 5, dans lequel la portion creuse comprend au moins une rainure.

9. Dispositif selon l'une quelconque des revendications 1 à 5, dans lequel la portion creuse comprend une pluralité de rainures

10. Dispositif selon la revendication 9, dans lequel chaque rainure est connectée de manière indépendante au système d'aspiration.

11. Dispositif selon l'une quelconque des revendications 1 à 4, dans lequel la portion creuse comprend une gorge annulaire.

## Patentansprüche

1. Vorrichtung zur Behandlung einer Augenkrankheit, Folgendes umfassend:
- einen Saugring (1), der ein proximales Ende umfasst, das dafür eingerichtet ist, am Auge eines Patienten anzuliegen, und das einen vertieften Abschnitt umfasst,
- ein Ansaugsystem (5), das mit dem vertieften Abschnitt verbunden ist, um die Luft im vertieften Abschnitt derart anzusagen, dass der Ring auf dem Auge des Patienten fixiert wird,
**dadurch gekennzeichnet, dass** das Ansaugsystem dafür eingerichtet ist, einen leichten Unterdruck zu erzeugen, der zwischen 80 und 150 Millimeter Quecksilber beträgt.

2. Vorrichtung nach Anspruch 1, bei der die Lage des vertieften Abschnitts auf dem Ring derart ist, dass wenn der Ring auf dem Auge positioniert ist, der vertiefte Abschnitt mit einer Zone des Auges in Kontakt ist, in der die Lederhaut mit einer feinen Bindehautmembran bedeckt ist.

3. Vorrichtung nach einem der Ansprüche 1 bis 2, bei der das Ansaugsystem dafür eingerichtet ist, einen Unterdruck zu erzeugen, der im Wesentlichen gleich 120 Millimeter Quecksilber ist.

4. Vorrichtung nach einem der Ansprüche 1 bis 3, bei der der Ring Folgendes umfasst: eine Öffnung, aus der die Hornhaut hervorsteht, sowie eine Trägeraufnahme, die dafür vorgesehen ist, ein Mittel zum Erzeugen von Ultraschall aufzunehmen.

5. Vorrichtung nach einem der Ansprüche 1 bis 4, bei der der vertiefte Abschnitt wenigstens eine Aussparung umfasst.

6. Vorrichtung nach einem der Ansprüche 1 bis 5, bei der der vertiefte Abschnitt mehrere Aussparungen umfasst.

7. Vorrichtung nach Anspruch 6, bei der jede Aussparung auf unabhängige Weise mit dem Ansaugsystem verbunden ist.

8. Vorrichtung nach einem der Ansprüche 1 bis 5, bei der der vertiefte Abschnitt wenigstens eine Rille umfasst.

9. Vorrichtung nach einem der Ansprüche 1 bis 5, bei der der vertiefte Abschnitt mehrere Rillen umfasst.

10. Vorrichtung nach Anspruch 9, bei der jede Rille auf unabhängige Weise mit dem Ansaugsystem verbunden ist.

11. Vorrichtung nach einem der Ansprüche 1 bis 4, bei der der vertiefte Abschnitt eine ringförmige Auskehlung umfasst.

## Claims

1. A device for treating an ocular pathology comprising
- a suction ring (1) including a proximal end adapted to abut the eye of a patient and comprising a hollow portion,
- an aspiration system (5) connected to the hollow portion to aspirate air in the hollow portion so as to fix the ring onto the patient's eye.
**characterized in that** the aspiration system is adapted to generate a low vacuum of between 80 and 150 millimetres of mercury.

2. The device according to claim 1 wherein the position of the hollow portion on the ring is such that when the ring is in position on the eye, the hollow portion is in contact with a region of the eye where the sclera is covered with a thin conjunctiva membrane.

3. The device according to any of claims 1 to 2, wherein the aspiration system is adapted to generate a vacuum of substantially 120 millimetres of mercury.

4. The device according to any of claims 1 to 3, wherein the ring comprises an opening from which the cornea of the eye emerges and a supporting cradle intended to receive ultrasound generating means.

5. The device according to any of claims 1 to 4, wherein the hollow portion comprises at least one cut-out.

6. The device according to any of claims 1 to 5, wherein the hollow portion comprises a plurality of cutouts.

7. The device according to claim 6, wherein each cut-out is connected independently to the aspiration system.

8. The device according to any of claims 1 to 5, wherein the hollow portion comprises at least one groove.

9. The device according to any of claims 1 to 5, wherein the hollow portion comprises a plurality of grooves.

10. The device according to claim 9, wherein each groove is connected independently to the aspiration system.

11. The device according to any of claims 1 to 4, wherein the hollow portion comprises an annular groove.
